Europäisches Patentamt

European Patent Office

Office européen des brevets

(11) Publication number: **0 510 851 A1**

# EUROPEAN PATENT APPLICATION

(21) Application number: **92303273.4**

(22) Date of filing: **13.04.92**

(51) Int. Cl.5: **A61M 25/01, A61M 39/00**

(30) Priority: **22.04.91 US 688176**

(43) Date of publication of application:
**28.10.92 Bulletin 92/44**

(84) Designated Contracting States:
**AT CH DE DK FR GB IT LI NL SE**

(71) Applicant: **ANGEION CORPORATION**
**13000 Highway 55**
**Plymouth, Minnesota 55441(US)**

(72) Inventor: **Ryan, William P.**
**15490 18th Avenue North 1512**
**Plymouth, Minnesota 55447(US)**
Inventor: **Savage, Steven D.**
**3101 Thurber Road**
**Brooklyn Center, Minnesota 55429(US)**

(74) Representative: **Parr, Ronald Edward R.E. Parr**
**& Co.**
**Colman House Station Road**
**Knowle Solihull West Midlands B93 0HL(GB)**

(54) Compression gasket for y-connector.

(57) A Y-connector (10) with a compression gasket (22) for compressing about a guidewire or catheter (13) when inserted into an inner chamber (50) of a Y-connector and compressed by a rim of a large knurled knob (32). When the large knurled knob is twisted with an inward force, the rim engages the gasket with inward compression, causing the center portion of the gasket to expand inwardly, thereby frictionally engaging against a guidewire or catheter. The inner expansion of the gasket is substantially equal about the inner circumference of the gasket, thereby providing a seal between the guidewire catheter and bodily fluids, such as blood. A guidewire clamp (21) is included at the end of a side arm extending from the Y-connector body.

FIG. 4

EP 0 510 851 A1

FIG. 2

The present invention relates to gaskets for sealing a catheter, guidewire or other elongate medical instrument with respect to a surrounding tubular housing, and to Y-connectors and other medical devices containing such gaskets, useful in catheter, guidewire and related medical procedures. The invention also relates to clamps for clamping a catheter, guidewire or other elongate medical instrument to a Y-connector or other medical device useful in catheter, guidewire or related medical procedures, and to Y-connectors and other medical devices including such clamps.

Prior art Y-connectors have given rise to problems of creating or maintaining adequate seals between a guidewire or catheter and the Y-connector, particularly the longitudinal chamber of the Y-connector through which the catheter or guidewire is inserted. If an adequate seal is not maintained between the guidewire or the catheter, body fluids leak out which is not only impractical, but is also unsanitary.

Prior art gaskets for Y-connectors were usually a piece of tubing, such as silicon tubing, but they did not provide a good gasket when compressed between the Y-connector and the guidewire or catheter. This not only allowed body fluids to leak out, but did not provide for easy sliding of the guidewire and, most dangerous, sometimes compressed the catheter so that it was unusable. However, replacement was often impractical since replacement of a catheter would usually result in considerable expense.

Where, in prior art devices, a Touhy Borst adapter was used with a Y-connector guidewires were often left unsecured in an unorderly fashion near the adapter of the Y-connector, sometimes allowing longitudinal movement of the guidewire in the adapter or kinking or bending of the guidewire immediately adjacent the adapter.

The present invention overcomes the disadvantages of the prior art by providing a Y-connector with a compression gasket which provides for more even compression about a guidewire or catheter, and also provides for sliding of the guidewire or catheter. Also provided is a guidewire clamp for positive positioning of a guidewire with reference to the Y-connector.

Accordingly, in a first aspect the present invention provides a gasket for sealing a catheter, guidewire or other elongate medical instrument (93) with respect to a tubular housing (38) surrounding said instrument,

characterised in that the gasket comprises a body (104) of resilient material having an axial passage (105) therethrough slidably to receive said medical instrument, said body being axially compressible whereby the cross-sectional dimension of the axial passage is diminished in size to an extent such that, in use, the passage closes about the medical instrument to provide a liquid-tight seal between said body and the medical instrument.

In a second aspect the invention provides a medical device,

characterised in that it comprises a catheter, guidewire or other elongate medical instrument (93);

a tubular housing (38) surrounding said instrument and disposed substantially co-axial to it; and

a gasket according to the first aspect of the invention disposed in the housing and substantially co-axial to it, the passage of the gasket accommodating the instrument;

means associated with the tubular housing to apply an axial compressive force to the gasket thereby to cause the cross-section of the passage to contract in size and thereby to close about the medical instrument and to seal the instrument in the housing.

For the sake of convenience the medical devices of the present invention are referred to below as "Y-connectors". However, the invention is not limited to such connectors or indeed to medical devices having a configuration in the shape of a "Y" but includes medical devices in general which include a catheter, guidewire or other elongate medical instrument surrounded by a tubular housing. If the housing includes, or is associated with, one or more side or branch arms or lumens or is otherwise bifurcated or branched, it can be of a configuration other than a "Y" configuration.

In a preferred form the present invention provides a Y-connector for medical procedures, such as cardiology and particularly angioplasty. The Y-connector or other similar medical device is conveniently one having a Touhy Borst adapter. In this preferred form the use of a gasket of the invention equalizes compression about, for example, a guidewire or catheter so that the guidewire or catheter can be easily slid through the longitudinal opening of the Y-connector, and yet at the same time, block seepage of any body fluids, such as blood, past the gasket.

Single and double Touhy Borst Y-connectors are used in angioplasty procedures. They facilitate easy insertion and sealing about a flimsy medical device, such as an angioplasty catheter or guidewire, and provide for subsequent fluid seal by turning of a knob located at the proximal end. The distal end can, for example, include a rotating swivel connector including a seal, for example, a quad ring seal.

Preferred forms of the medical devices of the present invention are now described with particular reference to the use of devices in the form of Y-connectors.

According to one embodiment of the present invention, there is provided a Y-connector with a Touhy Borst adapter and including the components; namely, a body Touhy Borst, a rotating connector, an hour glass configured compression gasket, a side port, and a guidewire clamp on a side arm. Each component is made, for example, of medical grade polymer materials.

The body is the main structure of the Y-connector, and is, for example, an injection-molded polycarbonate material with two internal lumens configured in a Y-orientation. The straight-through part of the Y is used for insertion of a medical instrument and is connected with a Touhy Borst adapter. The branch of the Y contains a female luer connector that can be used for a number of functions, for example an additional device, injection of flushing medium such as saline or injection of contrast medium such as Renographin (Trade Mark).

A highly elastomeric polymer compression gasket having a small central through hole positions in the adapter and can be deformed under longitudinal pressure. The cap of the Touhy Borst adapter is threaded on the rear of the adapter and used to put axial pressure on the compression gasket deforming it around a medical instrument such as a guidewire inserted in the hole and thus providing a seal. If no such device is present, the hole collapses on itself and seals against loss of any fluids.

A further component is a rotating connector which is used to connect the Y-connector to a mating medical device, such as a guiding catheter in an angiography procedure. The rotating connector contains a set of internal luer threads to allow for locking of the Y-connector onto another standard luer connection. A quad seal with both axial and radial compression provides a seal between the body and the rotating connector. Any other suitable seal could be utilized which provides axial and radial compression. Rotation is desirable so that the orientation of the Y-connector can be changed relative to the mating medical device and operating room environment. This is important in positioning the flushing port relative to the patient.

To accommodate the various requirements of interventional cardiology procedures, the Y-connector with Touhy Borst can be configured in different configurations, different, for example, with respect ot the number of Touhy Borst connectors available on each device and the availability of a side arm. Multiple Touhy Borst adapters are used for interventional cardiology procedures in which two devices must be inserted and positioned in the vascular system. The side arm adapter is required for those procedures in which the guidewire is not coaxial with the center line of the balloon catheter, but lies along the outside of the device. The use of the side arm allows the guidewire position to be fixed independent of the interventional catheter which has the advantage of exchanging catheters without the use of an exchange wire.

According to one embodiment of the present invention, there is provided a compression gasket for a Y-connector, which is uniquely configured so that under compression, the inner circumference compresses equally about a guidewire or catheter. The compression gasket includes a cylindrical-like member with an inner rim at a center portion, and an outer circumference with a hour glass type configuration at a center portion. Optional vertical reinforcing members extend about the outer circumference between the top and bottom edge, and along the outer circumference of the hour glass configuration. The compression gasket is configured so that equalized pressure between the bottom and top circumferential edges provide that the inner circumference is displaced inwardly and substantially equally about a guidewire or catheter.

A guidewire clamp secures at the end of a side arm for securement and fixation by snap engagement of a guidewire to the Y-connector.

Preferred forms of the invention, given by way of example, have one or more of the following features (a) to (g) below.

(a) a silicon rubber compression gasket for a Y-connector which can be used in any Y-connector type structure.

(b) a silicon rubber compression gasket for a Y-connector which equally compresses about a guidewire or catheter, enabling the guidewire or catheter to be easily slid through the compression gasket, the compression gasket also providing a pressure seal against reverse pressure of body fluids, such as blood, trying to pass out through the longitudinal lumen of the Y-connector.

(c) a swivel connector with a quad elastomeric seal which utilizes both radial and axial compression.

(d) an ergonomically designed shaft to fit in one's hand, a larger thumb wheel and a large through lumen.

(e) a quad elastomeric seal between the body and the rotating connector.

(f) a large lumen up to or greater than 9 French. The swivel and cap are snap-on components providing fewer moving components for ease of operation. The snap-on cap prevents the cap from separating from the body.

(g) a frictionally engaging guidewire clamp at one end of a side arm.

There are now described, by way of example and with reference to the accompanying drawings, preferred embodiments of the gasket, Y-connector and clamp aspects of the invention.

In the drawings:

FIG. 1 illustrates a plan view of the Y-connector including a Touhy Borst adapter;

FIG. 2 illustrates a cross-sectional view of the Y-connector of FIG. 1 showing the Touhy Borst adaptor separate from the Y-connector;

Fig. 3 illustrates a cross-sectional view of the Y-connector of FIG. 1 showing the Touhy Borst adapter in place on the Y-connector;

FIG. 4 illustrates a perspective view of the compression gasket;

FIG. 5 illustrates a side view of the gasket of FIG. 4;

FIG. 6 illustrates a cross-sectional view taken along line 6-6 of FIG. 5;

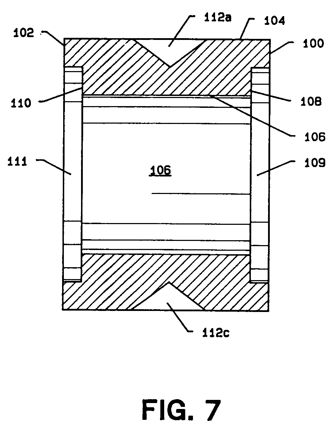

FIG. 7 illustrates a cross-sectional view taken along line 7-7 of FIG. 5;

FIG. 8 illustrates alignment of the compression gasket in the body of the Y-connector;

FIG. 9 illustrates a cross-sectional view taken along line 9-9 of FIG. 10;

FIG. 10 illustrates the sealing operation of a guidewire or other medical instrument by the compressed compression gasket;

FIG. 11 illustrates an exploded view of the clamp;

FIG. 12 illustrates the clamp engaging the side arm of the Y-connector; and

FIG. 13 illustrates a guidewire or medical device frictionally engaged by the clamp.

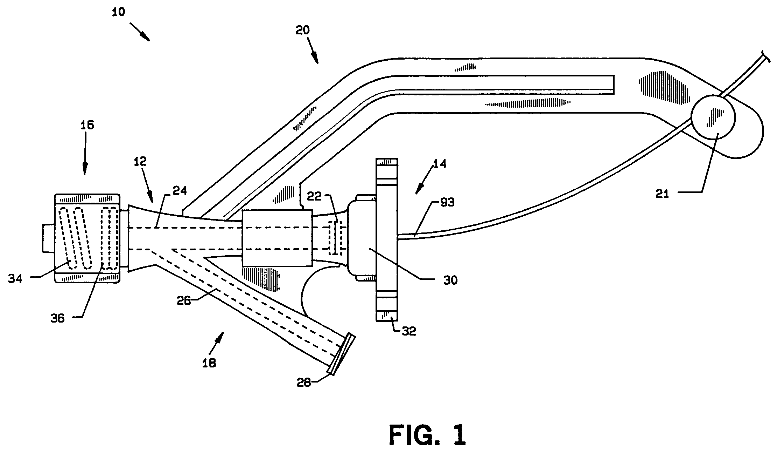

With reference to FIG. 1 the Y-connector (10) comprises as major components: a body (12), a Touhy Borst adapter (14), a rotatable connector (16) (referred to also below as a "rotating connector"), a side port (18), a side arm (20), a guidewire clamp (21), and a compression gasket (22), all of which are described later in detail. Each component is made, for example, of medical grade polymer material. The body (12) is the main structure of the Y-connector (10), it is of injection-molded polycarbonate material, and has two lumens (24 and 26) configured in a Y-orientation. The straight-through part of the Y, which contains lumen (24), is used for insertion of a circular medical device and is connected to the Touhy Borst adapter (14). The branch of the Y having lumen (26) contains a female luer connector (28) that can be used for a number of functions, for example for an additional device, injection of flushing medium such as saline, or injection of contrast medium such as Renographin.

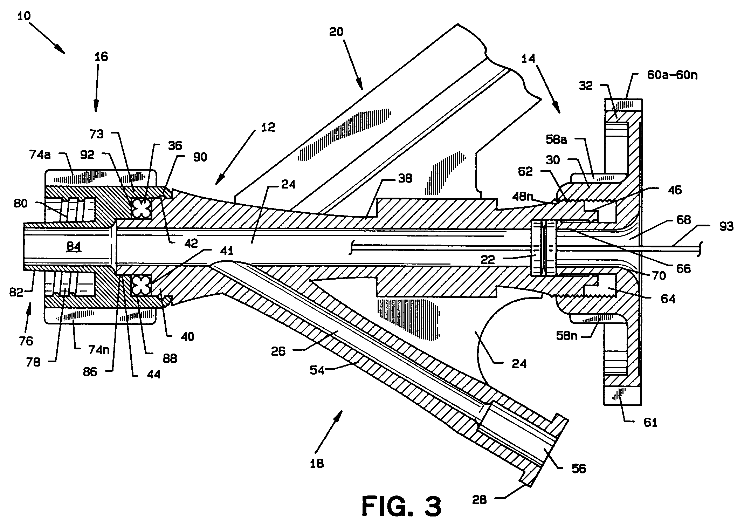

Compression gasket (22) can be deformed under pressure; it has a through hole and rests in a seat. The cap (30) of the Touhy Borst adapter is threaded and is used to apply pressure longitudinally on the compression gasket (22) deforming it inwardly around the inserted medical device, such as a guidewire, thus providing a seal. If no device is present, the hole collapses on itself and seals against any loss of fluids. The cap (30) includes a large thumb wheel knob (32) for any movement, torquing and locking. Rotating connector (16) is used to connect the Y-connector (10) to a mating medical device, such as a guiding catheter, and it contains a set of internal luer threads (80) to allow for locking of the Y-connector (10) onto another standard luer connection. In addition, rotation is provided about a quad O-ring and/or O-ring (36), as later described in detail with reference to FIG. 3, so that the orientation of the Y-connector (10) can be changed relative to the mating medical device. This is important in positioning the flushing port relative to the patient.

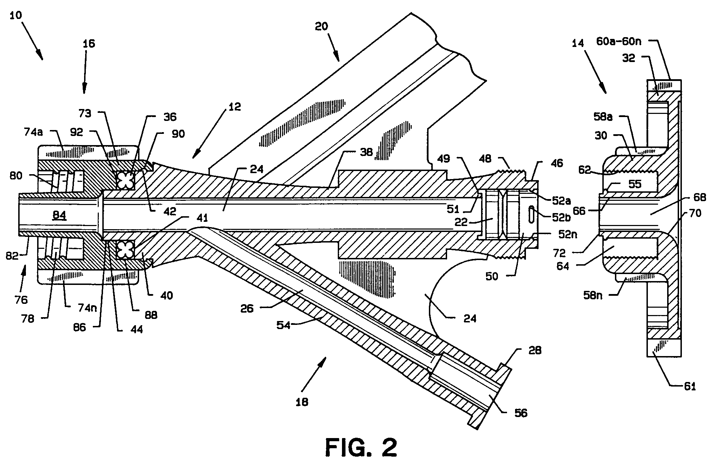

In FIG. 2 lumen (24) aligns longitudinally along the ergonomically sculptured main body portion (38) of the body (12). Body portion (38) has a radiused shoulder (40) with an adjoining flat doughnut-like annular surface (41), an annular raised ring (42) and a lesser radiused shoulder (44) and aligns concentrically along the longitudinal center line of the lumen (24) at the left end of the body (12). The O-ring (36) (for example a quad or quadruple O-ring as shown in FIGS. 2 and 3) frictionally engages the lesser radiused shoulder (44).

At the opposite end of body (12) a radiused shoulder (46) having threads (48) aligns concentrically with the longitudinal center line of the lumen (24). A radiused cavity (50) (FIG. 2) aligns concentrically with the lesser radiused lumen (24) at the right end of the body (12) and intersects a recessed seat (49). An annular lip (51) extends outwardly to the right from the recessed seat (49) to accommodate and engage the compression gasket (22) as later described in detail. The flexible plastic compression gasket (22) aligns in the radiused cavity (50) with the recessed seat (49). Protrusions (52a - 52n) at the mouth of the cavity (50) act as keepers to capture the compression gasket (22) in the cavity (50).

With reference to side port (18), lumen (26) aligns within the extension body (54), and intersects and connects with the lumen (24). A cavity (56) in conjunction with the female luer connector (28) aligns concentrically with the lumen (26). Protrusions (52a - 52n) also serve to capture a snap ring (55) on the end of the tubular extension of the Touhy Borst cap (30) to keep the thumb wheel knob (32) loosely coupled to the body (12).

The Touhy Borst adapter (14) aligns with and threadingly engages the main body (12) and includes a thumb wheel knob (32), pluralities of gripping ribs (58a - 58n) and (60a - 60n) and an index tab or rib (61). The cap (30) includes internal threads (62) which align and mate with the threads (48) of the body (12). An annular cavity (64) aligns concentrically with the center line of the cap (30) and is bounded by the internal threads (62) and by a concentric tubular extension (66) which extends to the left from the thumb wheel knob (32) and includes a passage hole (68) with a flared opening (70). An annular lip (72) extends from the tubular extension (66) to engage and accommodate the compression gasket (22).

The rotating connector (16) includes a round main body (73) having a plurality of external gripping ribs (74a - 74n) around its outer circumference. A male luer (76) extends longitudinally from the main body (73) through a cavity (78). Raised threads (80) line the cavity (78). The cavity (78), raised threads (80), a ramped cylinder section or male section (82), and surrounding main body (73) form the male luer (76). The passage way (84) formed by the ramped cylinder section (82) aligns concentrically with radiused cavities (86) and (88) and also with a rounded annular groove (90) which is located off-center to the right of the cavity (88). Cavity (88) includes a flat doughnut-like annular surface (92) adjacent to the radiused portion of the cavity (88).

With reference to FIG. 3, the Touhy Borst adapter (14) is mated to the body (12) by engagement of internal threads (62) and (48). The tubular extension (66) of the adapter (14) is thus forced against the compression gasket (22) to cause the effective size of an internal hole in the gasket to decrease in diameter and seal against a guidewire (93) as it is pressed against the recessed seat (49) and the annular lip (51), as later described in detail.

The rotating connector (16) snappingly engages the left end of the body (12). The cavity (86) accommodates the radiused shoulder (44) of the body (12). The quad O-ring (36) seals the rotating connector (16) to the body (12) by sealing of its external surfaces against the lesser radiused shoulder (44) of the body (12), the doughnut-like annular surface (41) of the body (12), the circumference of the cavity (88) of the rotating connector (16) and the flat doughnut-like annular surface (92) of the rotating connector (16). The annular raised ring (42) of the body (12) snappingly engages the rounded annular groove (90) of the rotating connector (16) to hold the body (12) and the rotating connector (16) in alignment and to maintain pressure against the quad O-ring and/or O-ring (36).

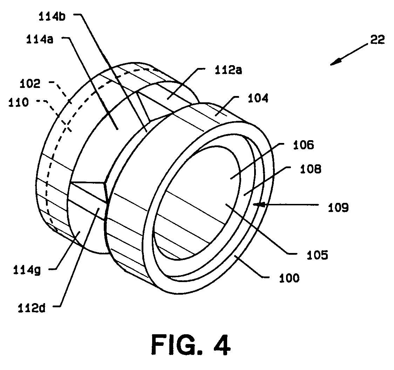

FIG. 4 illustrates a perspective view of the compression gasket (22), which can be made of silicon rubber or any other suitable, compressible material which has a memory to return to its original geometrical shape when not compressed. The compression gasket (22) includes a right hand edge (100) and a corresponding and opposed left hand edge (102). The outer circumferential wall (104) is shaped in an hour glass configuration. The particular geometrical configuration of the outer circumferential wall (104) is that as illustrated in the Figure, but it is within the teachings of the present invention to vary the geometrical configuration as to the exact hour glass configuration. The gasket has a cylindrical aperture or other inner hole (105) aligned in the inner circumference (106) and includes adjacent right hand and left hand annular rims (108, 110) offset, respectively, from the right hand edge (100) and the left hand edge (102). The inner circumference (106) is for frictional engagement against a guidewire, catheter or medical device as later described in detail. Optional vertical ribs (112a - 112d) are provided for supporting the right hand edge (100) with respect to the left hand edge (102). These vertical ribs cause the compression gasket (22), when compressed, to internally close in a square or other rhombic configuration about a medical device. Cavity (109) is the space between the right hand annular rim (108) and the plane of the right hand edge (100). A similar, opposing, cavity (111) is the space between the left hand annular rim (110) and the plane of the left hand edge (102). Cavities (109 and 111) are to accommodate the annular lip (72) of the Touhy Borst adapter (14) and the annular lip (51) of the body (12), respectively, for compressional alignment of the compression gasket (22) between those lips.

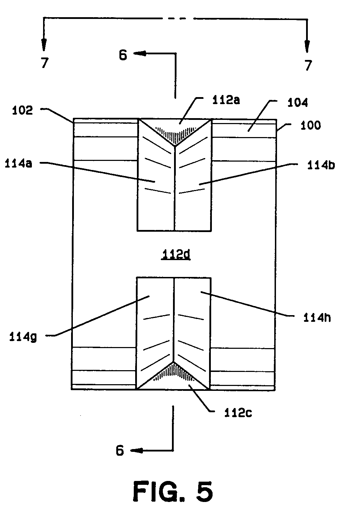

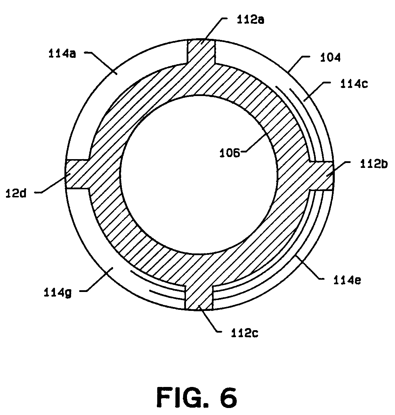

In FIG. 5 and FIG. 6 angled walls (114a and 114b) align between the ribs (112a and 112d) and the outer circumferential wall (104) as illustrated, and angled walls (114c - 114d), (114e - 114f) and (114g - 114h) align between the ribs (112a - 112d) and about the circumferential wall (104) in a similar fashion.

## MODE OF OPERATION

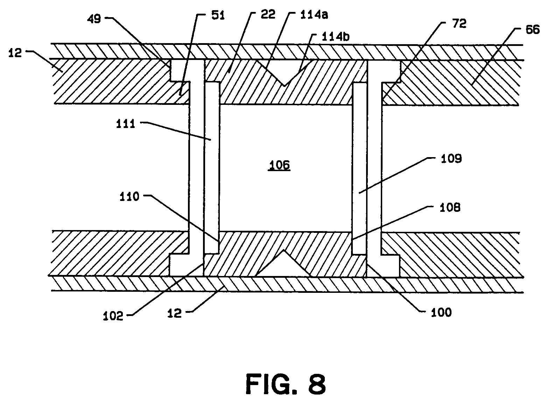

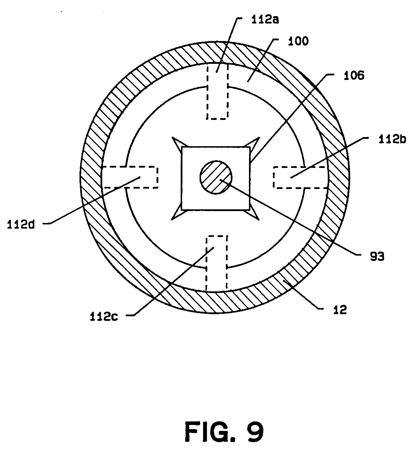

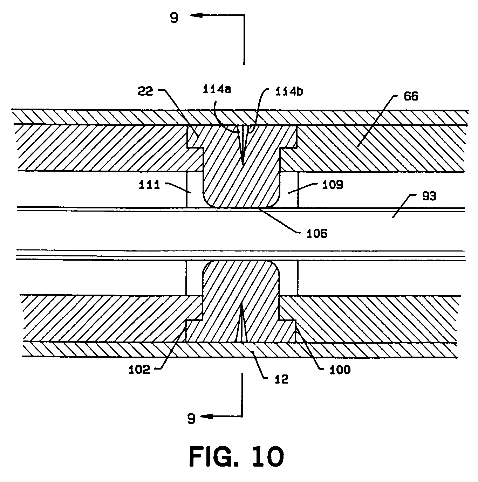

FIGS. 8 - 10 illustrate the mode of operation where the compression gasket (22) is compressed in the radiused cavity (50) from each side to seal about the guidewire (93). Cavities (109 and 111) in the compression gasket (22) are in alignment for accommodation, respectively, by the annular lip (72) of the Touhy Borst adapter (14) and annular lip (51) of the body (12). As the Touhy Borst adapter (14) is advanced on the body (12), the wall of the inner circumference (106) is forced inwardly an downwardly toward the guidewire (93) (FIG. 9). The wall of the inner circumference (106) assumes rhombic to square to round shape (that is, in succession rhombic, square and round shape) because of influence by the ribs (112a - 112d) during initial compression, and during final compression assumes a circular seal about the guidewire (93). As illustrated in FIG. 10, angled walls (114a - 114B) (and corresponding unillustrated angled walls (114c - 114d, 114e - 114f and 114g - 114h)) fold inwardly towards their like mirror surfaces to aid in full inward movement of the inner circumference to cause sealing of the guidewire (93). A sufficient fluid seal is maintained while still maintaining the ability of the guidewire (93) to move to and fro in sliding engagement.

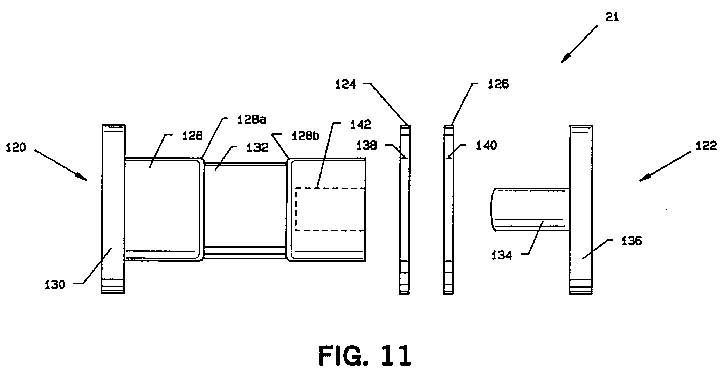

With reference to FIG. 11, clamp (21) snappingly affixes a guidewire to the side arm support (20) as as illustrated in FIG. 1. Guidewire clamp (21) incudes left hand and right hand member (120, 122) which mutually engage each other. Flexible plastic discs (124 and 126) align over and about the clamp members (120 and 122). A multi-radius cylindrical shaft member (128) extends perpendicularly from a disc shaped head (130) and includes a centrally located wide recessed groove (132) having edges (128a and 128b) which are rounded or ramped to transition between the general large radius of the shaft (128) down to the wide groove (132). The right clamp member (122) includes a cylindrical shaft member (134) extending perpendicularly from a disc shaped head (136). The flexible plastic discs (124 and 126) include central holes (138 and 140) for engagement over the cylindrical shaft member (128), and they are cemented to the heads (130 and 136), respectively. A cylindrical hole (142) in the shaft (128) frictionally engages the cylindrical shaft (134) to mate the left and right clamp members (120 and 122).

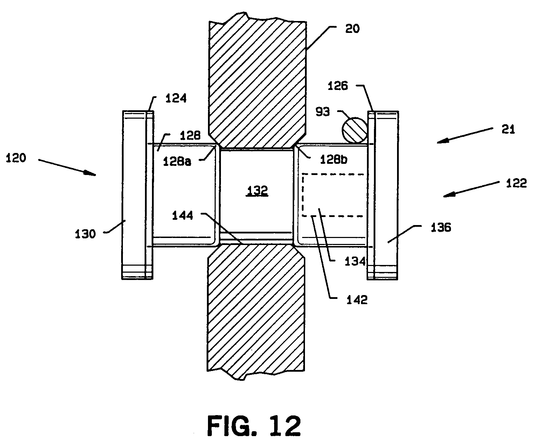

FIG. 12 illustrates the left and right clamp members engaging the side arm (20) of the Y-connector through beveled hole (144). The groove (132) frictionally and loosely engages the periphery of hole (144) to maintain the clamp (21) in a centrally aligned position with respect to the side arm (20) and allow a guidewire (93) to be readily positioned at either side of the side arm (20) and between the side arm and either of the flexible plastic discs (124 and 126).

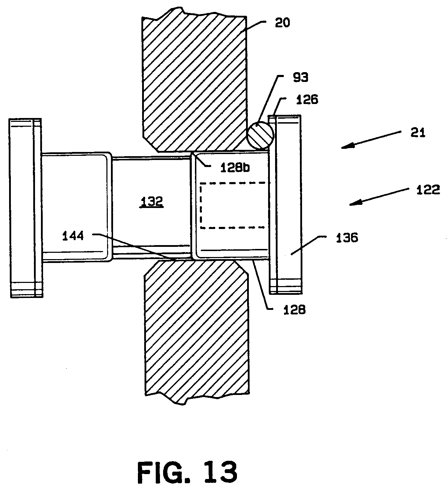

FIG. 13 illustrates the mode of operation of the guidewire (93) or other medical device frictionally engaged and secured between the side arm (20) and the flexible plastic disc (126) and adjacent disc shaped head (136), where the entire guidewire clamp (21) hasbeen forcibly positioned to the left to effect the securement of the guidewire to the side arm (20). During positioning, the hole (144) disengages from alignment or engagement with the groove (132) of the shaft (128) by action of the ramp (128b) against the beveled edges of the hole (144) to cause the wider radius portion of the cylindrical shaft member (128) to firmly engage the hole (144) for positive securement of the guidewire clamp (21) and guidewire with respect to the side arm (20).

The terms "hour glass configuration" and "hour glass shape" as used herein refer to configurations or shapes having a waisted portion or other portion of constricted width separating or disposed between two other portions of greater width. An example of an hour glass configuration is a dumbbell.

The term "gasket" as used herein refers to seals or obturations suitable for stopping up a tube, and includes seals of a plug-like nature. The gasket, conveniently of integral form, can be, for example, of a silicon rubber or other elastomer having high resilience.

## Claims

1. A gasket for sealing a catheter, guidewire or other elongate medical instrument (93) with respect to a tubular housing (38) surrounding said instrument,

   characterised in that the gasket comprises a body (104) of resilient material having an axial passage (105) therethrough slidably to receive said medical instrument, said body being axially compressible whereby the cross-sectional dimension of the axial passage is diminished in size to an extent such that, in use, the passage closes about the medical instrument to provide a liquid-tight seal between said body and the medical instrument.

2. A gasket according to Claim 1, wherein said substantially cylindrical periphery is of an hour glass shape.

3. A gasket according to Claim 1 or 2, wherein the ends (100, 102) of the body (104) have formed therein recesses (109, 111) to receive, respectively, members (14/72) and (12/51) adapted to transmit to the gasket said compressive force.

4. A gasket according to Claim 2 or 3, wherein the hour glass shape is provided by one or more circumferential grooves or channels (114) to facilitate closure of the passage about the medical instrument.

5. A gasket according to Claim 4, wherein there are two or more grooves or channels disposed longitudinally with respect to each other and separated by co-axial ribs (112).

6. A gasket according to any of the preceding claims, wherein the tubular housing (38) is a Y-connector.

7. A medical device, characterised in that it comprises a catheter, guidewire or other elongate medical instrument (93);

a tubular housing (38) surrounding said instrument and disposed substantially co-axial to it; and

a gasket as claimed in any of the preceding claims disposed in the housing and substantially co-axial to it, the passage of the gasket accommodating the instrument;

means associated with the tubular housing to apply an axial compressive force to the gasket thereby to cause the cross-section of the passage to contract in size and thereby to close about the medical instrument and to seal the instrument in the housing.

8. A medical device according to Claim 7, wherein, in the absence of the axial compressive force, the gasket is slidable within the housing and when the compressive force is applied to the gasket the cylindrical periphery thereof expands so as to provide a liquid-tight seal between said periphery and the tubular housing.

9. A medical device according to Claim 7 or 8, wherein the tubular housing comprises a Y-connector.

10. A compression gasket for a Y-connector (10) comprising:

    a. a geometrically configured cylinder (220) including a top edge (100) and a bottom edge (102);

    b. an hour glass configured outer circumference (104);and

    c. a rim (108,110) extending inwardly from an inner circumference (106).

11. In combination, a Y-connector and a compression gasket, comprising :

    a. a Y-connector (10) including a space between an inner rim of a longitudinal lumen and a base of a knurled knob; and

    b. a compression gasket including a geometrically configured cylinder (22) including a top edge (100) and a bottom edge (102), an hour glass configured outer circumference (104), and rim (108, 110) extending inwardly from an inner circumference.

FIG. 1

**FIG. 2**

FIG. 3

FIG. 4

EP 0 510 851 A1

FIG. 5

FIG. 6

EP 0 510 851 A1

112a

104

102

100

110

108

106

111

106

109

106

112c

**FIG. 7**

FIG. 8

FIG. 9

FIG. 10

FIG. 11

FIG. 12

FIG. 13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 267 584 (CORDIS CORP)<br>* abstract; figures * | 1,6-9 | A61M25/01<br>A61M39/00 |
| X | DE-C-3 834 600 (B BRAUN MELSUNGEN AG)<br>* column 6, line 14 - line 20; figures * | 1,6-9 | |
| X | DE-U-8 619 671 (STERIMED GMBH)<br>* page 3, line 25 - page 4, line 6; figures * | 1,7,8 | |
| A | EP-A-0 343 953 (NEWGARD) | | |

-----

| | | | TECHNICAL FIELDS SEARCHED (Int. Cl.5 ) |
|---|---|---|---|
| | | | A61M<br>F16J |

The present search report has been drawn up for all claims

| Place of search | Date of completion of the search | Examiner |
|---|---|---|
| THE HAGUE | 07 JULY 1992 | CLARKSON P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)